# EUROPÄISCHE PATENTANMELDUNG

(11) **EP 4 342 504 A1**
(43) Veröffentlichungstag der Anmeldung: **27.03.2024**
(21) Anmeldenummer: 23193674.1
(22) Anmeldetag: 28.08.2023
(51) Int. Cl.: A61M 1/06, G16H 20/60, G16H 40/20

(54) **VERFAHREN UND GERÄT ZUR BEHANDLUNG VON MUTTERMILCH**

(30) Priorität: 21.09.2022 DE 102022124250
(71) Anmelder: Olle Larsson Holding AG, 6300 Zug (CH)
(72) Erfinder: LARSSON, Michael, 6300 Zug (CH)
(74) Vertreter: Grünecker Patent- und Rechtsanwälte PartG mbB

(57) **Zusammenfassung**

Die vorliegende Erfindung betrifft ein Verfahren und ein Gerät zur Behandlung von Muttermilch, bei dem eine Brustpumpe an die weibliche Brust angelegt wird und die Brustpumpe betrieben wird, um Muttermilch abzupumpen und in einen Behälter zu überführen. Der Behälter wird geschlossen, wobei der Behälter mit einer den Behälter identifizierenden Kennung versehen ist oder wird. Eine den Behälter identifizierende wird generiert und dem Behälter zugeordnet. Zur Vermeidung einer Beeinträchtigung der Muttermilch wird diese in dem Behälter zur Verfütterung verfügbar gemacht. Das erfindungsgemäße Gerät zur Behandlung von Muttermilch hat eine Aufnahme für einen Muttermilch enthaltenen Behälter und eine Schließeinrichtung zum Verschließen des Behälters, eine Einrichtung zur Behandlung der in dem Behälter enthaltenen Muttermilch zur Verbesserung der Haltbarkeit der Muttermilch, eine Einrichtung zum Bestimmen der Menge der in dem Behälter enthaltenen Muttermilch, eine Leseeinrichtung zum Auslesen einer an dem Behälter vorgesehenen Kennung oder Anbringeinrichtung zum Anbringen einer Kennung an dem Behälter und eine Schnittstelle zum Übermitteln von Informationen an einen Onlinespeicher, die die Kennung und die ermittelte Menge an Muttermilch kennzeichnen.

## Beschreibung

Die vorliegende Erfindung betrifft ein Verfahren zur Behandlung von Muttermilch sowie ein Gerät zur Behandlung von Muttermilch.

Früh- und Frühstgeborene werden üblicherweise mit Muttermilch ernährt. Dabei kann die von der vorliegenden Erfindung in den Blick genommene Muttermilch eine Milch sein, wie von der das Früh- bzw. Frühstgeborene gebärenden oder aber einer anderen Spenderin für Muttermilch abgegeben wird.

Die vorliegende Erfindung will ein Verfahren und ein Gerät angeben, die das hygienisch unbedenkliche und einfache Spenden von Muttermilch ermöglichen.

Bei dem erfindungsgemäßen Verfahren wird mit einer Brustpumpe, die an die weibliche Brust angelegt wird, Muttermilch abgepumpt und in einen Behälter überführt. Der Behälter ist dabei fluidisch unmittelbar mit der Brustpumpe verbunden. So wird die abgepumpte Muttermilch durch den Betrieb der Brustpumpe unmittelbar in den Behälter überführt. Damit wird vermieden, dass durch mehrfaches Umfüllen von Muttermilch diese verlorengeht bzw. sich hygienisch bedenkliche Zustände ergeben, die die Muttermilch unbrauchbar machen können. Ein Behälter wird dabei vorzugsweise steril bzw. keimarm verpackt, und in dieser Verpackung der Spenderin der Muttermilch zur Verfügung gestellt und in diesem Zustand mit der Brustpumpe fluidisch verbunden. Dazu kann beispielsweise ein flexibler Beutel mit dem Brustschild fluidisch verbunden, bevorzugt unmittelbar daran befestigt und mit dem Brustschild gekoppelt werden. Zu bevorzugen sind kurze Leitungswege zwischen der Brust und dem Behälter, so dass am Ende der Spende der Muttermilch möglichst das gesamte abgegebene Volumen an Muttermilch in den Behälter überführt ist und nur zu einem sehr geringen Restvolumen in dem Leitungsweg zwischen der weiblichen Brust und dem Behälter verbleibt. Der Leitungsweg in einen durch den Beutel gebildeten Behälter innerhalb der Brustpumpe kann ganz überwiegend durch beispielsweise tüllenförmige Wandsegmente des Beutels selbst gebildet sein, die den Eingang in den Beutel bilden.

Der in dieser Weise befüllte Behälter wird verschlossen. Auch hierbei ist auf keimfreie bzw. sterile Bedingungen zu achten. Der Verschluss kann ein Schraubverschluss sein, der lediglich an solchen Flächen gehandhabt wird, die auf der Außenseite des Behälters vorgesehen sind und nicht mit der Muttermilch in Berührung kommen. Ein solcher Verschluss wird üblicherweise zusammen mit dem Behälter in der sterilen bzw. keimfreien Verpackung der Spenderin zugeliefert. Der Behälter kann auch ein in einer sterilen Verpackung zugelieferter flexibler Beutel sein. Die Verwendung eines Beutels als Behälter hat mehrere Vorteile, auf die nachstehend noch eingegangen wird.

Der Behälter kann bereits im zugelieferten Zustand mit einer Kennung versehen sein, die den Behälter eineindeutig identifiziert. Der Behälter kann aber alternativ auch durch den Spender bzw. am Ort des Spenders mit einer entsprechenden Kennung versehen werden. Die Kennung wird mit zumindest einer Eigenschaft der in dem Behälter enthaltenen Milch in einem Online-Speicher abgespeichert. Die Eigenschaft der Milch kann dabei beispielsweise die Menge der in dem Behälter enthaltenen Milch sein. Auch qualitative Eigenschaften, wie der Zeitpunkt der Spende, die auf die Frische der Muttermilch hinweist, oder aber Ergebnisse der chemischen Analyse der gespendeten Muttermilch können Eigenschaft derselben sein. Auch etwaige der Muttermilch zugegebene Medikamente bzw. Nährstoffe, insbesondere in Form von Fortifiern können als Eigenschaft der Muttermilch in dem Online-Speicher und zu dem entsprechenden Behälter abgespeichert werden.

Entsprechendes gilt für die Dokumentation einer Behandlung der Muttermilch vor dem Verfüttern beispielsweise durch Dokumentation des oder der durchgeführten Behandlungsschritte und/oder der durch die Behandlung modifizierten Eigenschaft oder Eigenschaften der Muttermilch. Die Behandlung kann insbesondere das Filtern beispielsweise zum Entfernen von toxischen Inhaltsstoffen aus der Muttermilch, oder das Aufkonzentrieren umfassen. Beim Aufkonzentrieren wird üblicherweise ein gewisser Wasseranteil aus der Muttermilch entfernt.

Die Kennung ist dabei in der Regel eine maschinenlesbare Kennung, beispielsweise eine RFID-Kennung oder ein Barcode. Durch Einlesen der Kennung an einem Eingabegerät, beispielsweise einem PC, lassen sich aus dem Online-Speicher die dort hinterlegten Eigenschaften zu dem Behälter auslesen. So können die Eigenschaften der Muttermilch beispielsweise vor der Verfütterung der Muttermilch an ein Früh- der Frühstgeborenes überprüft werden.

Das Auslesen der Kennung erfolgt dabei zumindest an einer Übergabestelle, an der die in dem Behälter enthaltene Milch gelagert oder verbraucht wird. Eine solche Übergabestelle kann beispielsweise durch den Wareneingang an einem Krankenhaus gegeben sein, in dem das zu ernährende Früh- bzw. Frühstgeborene versorgt wird. Eine Übergabestelle kann aber beispielsweise auch der Eingang in eine Lagerhaltung sein, die zentral oder dezentral betrieben wird. Eine dezentrale Lagerhaltung kann beispielsweise auch am Ort der Spenderin erfolgen.

Das Auslesen der Kennung und gegebenenfalls der damit in dem Online-Speicher hinterlegten Informationen zu den Eigenschaften der in dem Behälter enthaltenen Muttermilch erlaubt eine angemessene Lagerung, einen zeitgerechten Verbrauch der Muttermilch sowie eine vorausplanende Verfütterung der Muttermilch an das Früh- bzw. Frühstgeborene. Erfolgt das Auslesen der Kennung an einer Übergabestelle, an welcher der Inhalt des Behälters verfüttert wird, so können die mit der Kennung ausgelesenen Eigenschaften automatisiert protokolliert und in Form eines Ernährungsprofils zu dem jeweiligen Früh- bzw. Frühstgeborenen zu Kontroll- bzw. Dokumentationszwecken abgespeichert werden.
Die Muttermilch wird dabei nach dem vorliegenden Verfahren üblicherweise unmittelbar aus dem Behälter direkt an das Früh- bzw. Frühstgeborene verfüttert. Der Behälter wird also zur Verfütterung der Muttermilch am Ort des Verfüttern verfügbar gemacht wird. So wird üblicherweise beim Verfüttern der Muttermilch eine fluidische Verbindung zwischen dem Behälter und dem Verdauungstrakt des Früh- bzw. Frühstgeborenen hergestellt. So wird der Behälter bevorzugt zum Verfüttern fluidisch mit einer Ernährungsonde verbunden. Diese befindet sich üblicherweise an dem Ende eines Schlauches, der zwischen der Ernährungssonde und dem Behälter vorgesehen und an den Behälter angeschlossen ist. Zwar sind die Organe des frühst- oder frühgeborenen Kindes und insbesondere sein Verdauungstrakt sind noch nicht ausgereift und können nicht von Geburt an die Gesamtheit seines Flüssigkeits- und Nahrungsbedarfs auf oralem Weg aufnehmen, so dass üblicherweise sofort nach der Geburt eine Infusion gelegt, über die eine parenterale Ernährung durchgeführt werden kann. Diese versorgt es mit Zucker, Proteinen, Fetten, Mineralstoffen und Vitaminen. Allerdings sollte es parallel dazu und so bald wie möglich Muttermilch gefüttert werden, um den Verdauungstrakt anzuregen. Muttermilch besitzt zahlreiche Eigenschaften, die für Frühgeborene von Vorteil sind, und wird vom nicht ausgereiften Darm viel besser vertragen und verdaut als künstliche Säuglingsnahrung. Hier setzt die Erfindung insbesondere an und will die kontrollierte Verabreichung von abgepumpter Muttermilch über eine kleine Ernährungssonde, die durch die Nase bis in den Magen geschoben wurde, sicherer und zuverlässiger machen. Die Kennung zu dem Behälter und ggf. das Abspeichern zumindest einer spezifischen Eigenschaft der Muttermilch erfolgt vorzugsweise in einer Art einer Blockchain, die die Dokumentation und Nachverfolgung der Muttermilch vom Abpumpen bis zum Verfüttern ermöglicht.

Muttermilch, die in einem zuvor erwähnten Verfahren zum Einsatz kommt, kann auch durch stillende Mütter als Spende für einen anderen Säugling als den eigenen abgegeben werden. Die vorliegende Erfindung will die Spendeaktivität solcher Spenderinnen als Dritte incentivieren und die sichere Spende von Muttermilch erleichtern. So wird mit der vorliegenden Erfindung ein Gerät zur Behandlung von Muttermilch vorgeschlagen, das eine Aufnahme für den die Muttermilch enthaltenden Behälter hat. Das Gerät hat ferner eine Schließeinrichtung zum Verschließen des Behälters. Das Verschließen erfolgt üblicherweise automatisiert. Besonders bevorzugt erfolgt das Verschließen durch Verschweißen eines flexiblen Beutels, der den Behälter ausformt. Das Gerät hat üblicherweise auch eine Einrichtung zur Behandlung der in dem Behälter enthaltenen Muttermilch zur Verbesserung der Haltbarkeit der Muttermilch. Eine solche Behandlung kann beispielsweise eine Pasteurisierung mittels UV-Bestrahlung sein. Das Gerät ist ferner angepasst ausgebildet, die Menge der in dem Behälter enthaltenen Muttermilch zu bestimmen. Diese Bestimmung kann durch Erkennen des in dem Behälter enthaltenen Volumens und/oder durch Wiegen erfolgen. Dabei wird üblicherweise das Eigengewicht des Behälters dem Gerät mitgeteilt, beispielsweise durch Auslesen einer an dem Behälter vorgesehenen Kennung, die auch Informationen zum Eigengewicht des Behälters enthält. So lässt sich die Menge der in dem Behälter enthaltenen Muttermilch exakt bestimmen. Neben einer Leseeinrichtung zum Auslesen der an dem Behälter vorgesehenen Kennung kann das Gerät auch eine Einrichtung zum Anbringen einer Kennung an dem Behälter aufweisen. Diese Einrichtung kann als Teil der Kennung Informationen zu den Eigenschaften der Muttermilch hinterlegen, die in dem Gerät ermittelt werden. Zusätzlich enthält das Gerät eine Schnittstelle zum Übermitteln von Informationen an einen Online-Speicher, wobei die Informationen die Kennung bzw. zumindest die ermittelte Menge an Muttermilch in dem Behälter kennzeichnen. Das Gerät kann ferner eine Geräteidentifikation haben, die mit der Kennung an der Schnittstelle zur Speicherung in dem Online-Speicher bereitgestellt wird. Eine solche Geräteidentifikation erlaubt die Zuordnung einer spezifischen Spende an Muttermilch zu einem spezifischen Gerät und damit zu einer spezifischen Spenderin. Über diese Geräteidentifikation bzw. Identifikation der Spenderin ist es möglich, Informationen über die Eigenschaft der gespendeten Muttermilch an die Spenderin zurückzuleiten und damit weitere Spenden zu incentivieren. Die Rückkopplung erfolgt dabei üblicherweise über ein Mobilgerät, welches von der Spenderin gehalten wird und das datenmäßig mit dem Online-Speicher verbunden ist oder wird, um Informationen zur Eigenschaft, beispielsweise zur Menge der gespendeten Milch an den Online-Speicher zu übermitteln. Damit ist eine engmaschige und einfache Betreuung der Spenderin gegebenenfalls auch Überwachung der Qualität der Muttermilch bei der Spenderin möglich. So können der Spenderin beispielsweise Ergebnisse einer chemischen Analyse der Muttermilch zurückgespielt werden, die auf eine bedenkliche Qualität der Muttermilch und/oder eine Krankheit bei der Spenderin, beispielsweise Mastitis, hinweisen. Sofern die Spenderin ein eigenes Kind stillt, erlaubt diese Art der Rückkopplung danach auch eine qualitative Überwachung der dem eigenen Säugling gegebenen Muttermilch.

Das Gerät ist üblicherweise auch angepasst ausgebildet zur Abgabe einer zeitlichen Angabe zusammen mit der Kennung an die Schnittstelle. Diese zeitliche Angabe erlaubt eine Bewertung der Frische der Milch. Die zeitliche Kennung steht für den Tag gegebenenfalls zusammen mit der Uhrzeit, zu der die Muttermilch abgepumpt und in den Behälter überführt wurde.

Die zuvor diskutierten Ausgestaltungen und Weiterbildungen des Geräts sind auch Weiterbildungen des erfindungsgemäßen Verfahrens und finden ihren Niederschlag in den abhängigen Ansprüchen 4 und 5.

Zum Verfüttern der Muttermilch wird der Behälter bevorzugt mit einem äußeren Druck beaufschlagt. Bei einem Behälter mit einer starren Außenwand wird der innere Druck in den Behälter eingebracht, so dass die in dem Behälter vorgesehene Flüssigkeitssäule aus dem Behälter herausgetrieben wird. Bei der bevorzugten Ausgestaltung des Behälters in Form eines flexiblen Beutels kann dieser mit einem hydrostatischen Druck von außen beaufschlagt werden. Ebenso ist es möglich, den flexiblen Behälter mechanisch mit einem Stempel oder dergleichen mit Druck zu beaufschlagen, um die in dem Beutel enthaltene Muttermilch auszufördern. Der äußere Druck wird dabei üblicherweise geregelt, so dass die ausgeförderte Menge an Muttermilch auf den gewünschten kontinuierlichen oder diskontinuierlichen Volumen- bzw. Massestrom eingestellt wird, der der gewünschten Rate zur Fütterung des Früh- bzw. Frühstgeborenen entspricht. Alternativ kann die Muttermilch auch nach Art einer Infusion durch Schwerkraft und unter Kontrolle des Volumenstromes wie bei einer Infusion aus dem Behälter abgegeben werden. Wesentlich ist in der Regel eine bedarfsgerechte gegebenenfalls auch konstante und kontrolliert ablaufende Abgabe der Muttermilch aus dem Behälter.

Die zuvor bereits diskutierte Möglichkeit einer chemischen Analyse bedingt das Ziehen einer Probe aus dem Behälter. Dazu hat ein den Behälter ausbildender flexibler Beutel üblicherweise eine speziell abgeteilte Kammer zur Probennahme und/oder ein wiederverschließbares, unter sterilen Bedingungen geschlossenes Einwegventil, welches lediglich zur Abgabe von Muttermilch angepasst ist. Alternativ kann der Behälter auch eine gesonderte Kappe zur Entnahme der Probe aufweisen. Solche gesonderten Kammern bzw. Verschlüsse sind üblicherweise an dem flexiblen Beutel mittels Verschweißen des flexiblen Folienmaterials des Beutels und/oder Verschweißen des Verschlusses ausgebildet, beispielsweise Abteilen einer Probenkammer durch zwei beabstandete Querschweißnähte im Bereich der tüllenförmigen Wandsegmente des Beutels, die den Eingang in den Beutel bilden.

Das Verfahren wird bevorzugt wie folgt durchgeführt: Eine Spenderin verbindet ihre Brustpumpe fluidisch mit dem Behälter, so dass beim Betrieb der Brustpumpe der Behälter mit der zu spendenden Muttermilch gefüllt wird.

Danach wird der Behälter von der Spenderin in das Gerät eingelegt, welches möglichst voll automatisiert die weiteren Schritte durchführt, nämlich den Behälter verschließt und ggf. eine Probenkammer abteilt, die Muttermilch zur Verbesserung der Haltbarkeit behandelt, die Menge der in dem Behälter enthaltenen Muttermilch bestimmt und die entsprechende in dem Behälter enthaltene Spende über die Kennung identifizierbar macht und diese Identifizierung in einen Online-Speicher, gegebenenfalls zusammen mit weiteren Informationen zur Eigenschaften der Muttermilch ablegt. Das Gerät kann dazu eine WLAN- oder LAN-Schnittstelle haben. Auch das Abspeichern erfolgt üblicherweise voll automatisiert, nachdem das Gerät eingerichtet worden ist.

Der Behälter wird dann beispielsweise einem Krankenhaus zugeliefert und dort gelagert. Die von dem Gerät ermittelten Informationen zu den Eigenschaften der Muttermilch werden parallel an das Mobilgerät der Spenderin gesandt. Am Wareneingang des Krankenhauses wird üblicherweise der Behälter gescannt, d.h. die Beutelkennung eingelesen bzw. in die Beutelkennung eingeschriebene Informationen zu den Eigenschaften der Muttermilch ausgelesen. Diese Informationen werden in einer zentralen Lagerhaltung des Krankenhauses abgespeichert. Gegebenenfalls wird eine Probe aus dem Behälter gezogen und diese chemisch analysiert. Auch das Analyseergebnis wird zu der Kennung und damit zu dem Behälter mit der Muttermilch abgespeichert. Insbesondere bei Auffälligkeiten erfolgt eine erneute Rückmeldung an die Spenderin.

Im Rahmen der Verfütterung kann die Muttermilch in dem Behälter nicht nur erwärmt, sondern auch mit Nährstoffkonzentrat, so genannten Fortifiern bzw. mit Medikamenten angereichert werden. Die Verfütterung erfolgt unmittelbar aus dem Behälter, indem auf den Behälter ein Druck aufgebracht wird.

Ersichtlich erlaubt das erfindungsgemäße Verfahren eine hygienisch einwandfreie Spende von Muttermilch, wobei das Verfahren die Muttermilch bestmöglich der späteren Verfütterung zuführt, da lediglich ein einziger Behälter zum Abpumpen und zum Verfüttern verwendet wird. Die Kennung erlaubt eine eineindeutige Identifizierung des Behälters und der darin enthaltenen Muttermilch, gegebenenfalls ergänzt um Informationen zu Eigenschaften, wie Menge, Alter und/oder chemische Inhaltsstoffe bzw. Modifikationen durch Fortifier und/oder Medikamente. Da die entsprechenden Informationen als Daten zu dem Behälter hinterlegt sind, können diese Daten beim Verfüttern in einen Ernährungsplan eingelesen werden, der die Ernährung des Früh- bzw. Frühstgeborenen dokumentiert.

Die Behälter werden üblicherweise den Spendern unter keimfreien bzw. sterilen Bedingungen zugeliefert und unter solchen Bedingungen aus einer sterilen Umverpackung entnommen, so dass zu jeder Zeit eine keimfreie bzw. -arme Handhabung der Muttermilch möglich ist, die sich bis zur Verfütterung durchzieht.

## Patentansprüche

1. Verfahren zur Behandlung von Muttermilch,
bei dem eine Brustpumpe an die weibliche Brust angelegt wird;
die Brustpumpe betrieben wird, um Muttermilch abzupumpen und in einen Behälter zu überführen;
der Behälter geschlossen wird,
eine den Behälter identifizierende Kennung generiert wird, die dem_Behälter zugeordnet ist oder wird und
der Behälter zur Verfütterung der Muttermilch verfügbar gemacht wird.

2. Verfahren nach Anspruch 1, **dadurch gekennzeichnet, dass**
der Behälter mit einer den Behälter identifizierenden maschinenlesbaren Kennung versehen ist oder wird;
dass zumindest eine der Eigenschaften der in dem Behälter enthaltenen Muttermilch zu der Kennung in einem Speicher abgespeichert wird und
dass die Kennung an einer Übergabestelle ein- bzw. ausgelesen wird, an der die in dem Behälter enthaltene Muttermilch gelagert und/oder verbraucht wird, um die zumindest eine Eigenschaft der in dem Behälter enthaltenen Muttermilch auszugeben.

3. Verfahren nach Anspruch 1 oder 2, **dadurch gekennzeichnet, dass** die Muttermilch beim Verfüttern unmittelbar aus dem Behälter herausgefördert wird.

4. Verfahren nach einem der vorherigen Ansprüche, **dadurch gekennzeichnet, dass** der Behälter zum Verfüttern an einen eine Ernährungssonde aufweisenden Schlauch angeschlossen wird.

5. Verfahren nach einem der vorherigen Ansprüche, **dadurch gekennzeichnet, dass** als Behälter ein flexibler Beutel verwendet wird.

6. Verfahren nach einem der vorherigen Ansprüche, **dadurch gekennzeichnet, dass** der Behälter nach dem Abpumpen der Muttermilch an ein Gerät übergeben wird, durch das der Behälter verschlossen wird, durch das die Muttermilch zur Verbesserung der Haltbarkeit in dem Behälter behandelt und die Menge der in dem Behälter enthaltenen Muttermilch bestimmt wird, durch das eine an dem Beutel vorgesehene Kennung ausgelesen oder der Behälter mit einer Kennung versehen wird und durch das zumindest die ermittelte Menge als Eigenschaft zusammen mit der Kennung zur Speicherung in dem Speicher abgegeben wird.

7. Verfahren nach Anspruch 6, **dadurch gekennzeichnet, dass** von dem Gerät eine Geräteidentifikation und/oder eine zeitliche Angabe zusammen mit der Kennung in dem Speicher zur Speicherung in dem Speicher abgegeben wird.

8. Verfahren nach einem der vorherigen Ansprüche, **dadurch gekennzeichnet, dass** der Behälter beim Verfüttern zum Ausfördern der Muttermilch mit einem äußeren Druck beaufschlagt wird und dass der äußere Druck zur Einstellung der ausgeförderten Menge an Muttermilch geregelt wird.

9. Verfahren nach einem der vorherigen Ansprüche, **dadurch gekennzeichnet, dass** der Muttermilch vor dem Verfüttern Nährstoffe und/oder Medikamente zugegeben werden.

10. Verfahren nach einem der vorherigen Ansprüche, **dadurch gekennzeichnet, dass** die Muttermilch vor dem Verfüttern behandelt wird, insbesondere gefiltert und/oder aufkonzentriert wird.

11. Verfahren nach einem der vorherigen Ansprüche, **dadurch gekennzeichnet, dass** aus dem Behälter eine Probe gezogen und analysiert wird und dass das Ergebnis der Analyse in dem Speicher der Kennung zugeordnet gespeichert wird.

12. Verfahren nach einem der vorherige Ansprüche, **dadurch gekennzeichnet, dass** zumindest eine zu der Muttermilch in dem Speicher abgespeicherte Eigenschaft an ein Mobilgerät gesendet wird, das als Mobilgerät der Spenderin der Muttermilch in dem Speicher hinterlegt ist.

13. Gerät zur Behandlung von Muttermilch mit einer Aufnahme für einen Muttermilch enthaltenen Behälter und einer Schließeinrichtung zum Verschließen des Behälters, einer Einrichtung zur Behandlung der in dem Behälter enthaltenen Muttermilch zur Verbesserung der Haltbarkeit der Muttermilch, einer Einrichtung zum Bestimmen der Menge der in dem Behälter enthaltenen Muttermilch, einer Leseeinrichtung zum Auslesen einer an dem Behälter vorgesehenen Kennung oder Anbringeinrichtung zum Anbringen einer Kennung an dem Behälter und einer Schnittstelle zum Übermitteln von Informationen an einen Speicher, die die Kennung und die ermittelte Menge an Muttermilch kennzeichnen.

14. Gerät nach Anspruch 13, **dadurch gekennzeichnet, dass** das Gerät angepasst ausgebildet ist, eine Geräteidentifikation und/oder eine zeitliche Angabe zusammen mit der Kennung an der Schnittstelle zur Speicherung in dem Speicher bereitzustellen.
